# EUROPEAN PATENT APPLICATION

(11) **EP 4 840 543 A1**
(43) Date of publication of application: **18.11.2026**
(21) Application number: 26169864.1
(22) Date of filing: 31.03.2026
(51) Int. Cl.: B01J 41/20, B01D 15/38, B01J 20/32, B01J 20/286, C07K 1/16

(54) **MIXED-MODE ANION-EXCHANGE CHROMATOGRAPHY MEDIUM, PREPARATION METHOD, AND APPLICATION THEREOF**

(30) Priority: 11.04.2025 CN 202510452902; 25.08.2025 CN 202511190025
(71) Applicant: Suzhou VDO Biotech Co., Ltd, Suzhou 215123 (CN)
(72) Inventor: Liao, Jiali, Suzhou (CN); Tang, Zhimin, Suzhou (CN); Zhang, Haoquan, Suzhou (CN); He, Honggang, Suzhou (CN); Kang, Haifan, Suzhou (CN); Li, Chang, Suzhou (CN); She, Yong, Suzhou (CN)
(74) Representative: Schwarz & Partner Patentanwälte GmbH

(57) **Abstract**

The present application provides a mixed-mode anion-exchange chromatography medium. The chromatography medium includes a ligand coupled to a matrix carrier. A structure of the ligand is represented by R1-R2-N(R3)-R4, wherein R1 is a substituted or unsubstituted phenyl group, and a substituent group of the substituted phenyl group is one or more selected from the group consisting of CH₃, CH₂CH₃, and CH₂CH₂CH₃; R2 is a substituted or unsubstituted hydrocarbon chain containing 2 to 6 carbon atoms, and a substituent group of the substituted hydrocarbon chain is one or more selected from the group consisting of O, CH₃, and CH₂CH₃; R3 is hydrogen or a hydrocarbon chain containing 1 to 2 carbon atoms; R4 is hydrogen or a hydrocarbon chain containing 1 to 2 carbon atoms; and the ligand is coupled to the matrix carrier via an amino group.

## Description

### TECHNICAL FIELD

The present application relates to chromatography media, in particular to a mixed-mode anion-exchange chromatography medium, a preparation method, and an application thereof.

### BACKGROUND

Chromatography technology is a crucial step in the purification of biomacromolecules in biological field. The chromatography medium is a core component of chromatography technology, and its performance directly impacts the purification efficiency and quality of target molecules. Therefore, the selection of the chromatography medium is critical. Common chromatography medium includes an ion exchange chromatography medium, a hydrophobic chromatography medium, a gel filtration chromatography medium, an affinity chromatography medium, etc. The ion exchange chromatography medium separates and purifies target substances based on the ion interactions, and is suitable for separating charged molecules, such as proteins and nucleic acids. The hydrophobic chromatography medium separates molecules based on hydrophobicity differences, and is suitable for separating and purifying hydrophobic molecules. The gel filtration chromatography medium, also known as molecular sieve chromatography medium, separates molecules based on molecular size differences. The affinity chromatography medium separates molecules based on specific affinities between the molecules, such as interactions between enzymes and inhibitors or between antibodies and antigens.

When selecting the chromatography medium, factors such as the properties of the sample, the characteristics of the target molecule, and the specific requirements of the purification process should be considered. For example, during the pretreatment stage, a medium with a large working capacity and a strong resistance to contamination is needed to handle a large volume and complex sample. During the refinement and purification stage, a medium with a high selectivity and a high resolution is needed to obtain a high-purity target product.

As the purification standards in the biopharmaceutical and biological products industries continue to rise, achieving efficient, rapid, and economical purification effects have become an important factor for evaluating the performance of the chromatography medium. The binding ability between the target molecule and the ligand is a critical factor affecting the purification effect. Chromatography medium with a weak binding ability may allow the target molecule to flow through the chromatography column directly without achieving purification. Moreover, for the purification of some high-salt samples, the conventional chromatography medium often shows issues such as decreased binding ability in high-salt environments. Therefore, before using the chromatography medium for purification, desalting treatment or dilution of the sample is typically required to reduce the salt concentration. Although such operations can achieve the equivalent purification effect, they inevitably extend the purification workflow and reduce the purification efficiency. Therefore, the binding ability and the salt tolerance are important consideration factors for evaluating the performance of the chromatography medium.

Although some salt-tolerant chromatography media with strong binding abilities have appeared on the market, they still have limitations when dealing with certain special samples. To achieve superior purification effect, the development of novel chromatography media holds significant value.

### SUMMARY

To address the issues existing in related art described above, an objective of the present application is to provide a mixed-mode anion-exchange chromatography medium, a preparation method, and an application thereof. The chromatography medium adopts a ligand possessing both hydrophobic and ion-exchange properties, thereby improving the salt tolerance and the binding ability to target molecules of the chromatography medium while satisfying loading requirements, and providing a more efficient and economical solution for sample purification.

To achieve the above objective, the present application provides the following technical solutions.

In a first aspect, the present application provides a mixed-mode anion-exchange chromatography medium. The chromatography medium includes a ligand coupled to a matrix carrier. A structure of the ligand is represented by R1-R2-N(R3)-R4, wherein:
R1 is a substituted or unsubstituted phenyl group, and a substituent group of the substituted phenyl group is one or more selected from the group consisting of CH₃, CH₂CH₃, and CH₂CH₂CH₃;
R2 is a substituted or unsubstituted hydrocarbon chain containing 2 to 6 carbon atoms, and a substituent group of the substituted hydrocarbon chain is one or more selected from the group consisting of O, CH₃, and CH₂CH₃;
R3 is hydrogen or a hydrocarbon chain containing 1 to 2 carbon atoms;
R4 is hydrogen or a hydrocarbon chain containing 1 to 2 carbon atoms; and
the ligand is coupled to the matrix carrier via an amino group.

In some embodiments, R3 and R4 are each hydrogen.

In some embodiments, R3 is a hydrocarbon chain containing 1 to 2 carbon atoms, and/or R4 is a hydrocarbon chain containing 1 to 2 carbon atoms.

Optionally, R3 is CH₃, and/or R4 is CH₃.

In some embodiments, R2 is an unsubstituted hydrocarbon chain containing 2 to 6 carbon atoms.

Optionally, R2 is CH₂CH₂.

In some embodiments, R1 is an unsubstituted phenyl group.

Optionally, the ligand is N,N-dimethyl-2-phenylethylamine or a salt thereof.

In some embodiments, R2 is a substituted hydrocarbon chain containing 2 to 6 carbon atoms.

Optionally, R2 is a substituted hydrocarbon chain containing 3 carbon atoms.

Optionally, hydrogen on a carbon atom bonded to R1 is substituted with an oxygen atom to form a C=O group.

Optionally, the ligand is 3-(dimethylamino)propiophenone or a salt thereof.

In some embodiments, the matrix carrier includes a polysaccharide-based matrix carrier.

In a second aspect, the present application provides a chromatographic column. The chromatographic column includes the mixed-mode anion-exchange chromatography medium as described above.

In a third aspect, the present application provides use of the above-described chromatography medium or the above-described chromatographic column in purifying a biomolecule.

In some embodiments, the biomolecule is a protein.

In some embodiments, the protein is an antibody, optionally, the antibody includes a monomer and an aggregate.

In a fourth aspect, the present application provides a method for purifying a biomolecule. The method includes: contacting a sample to be purified with the mixed-mode anion-exchange chromatography medium as described above or the chromatographic column as described above; and performing an elution to remove impurities and then collecting a purified target molecule, or purifying the target molecule using a flow-through mode.

Compared to existing technologies, the present application has the following beneficial effects:

In the present application, a novel ligand possessing both hydrophobic and ion-exchange properties is utilized to prepare the mixed-mode anion-exchange chromatography medium. It has been demonstrated through experimental data that the chromatography medium not only meets the loading requirements, but also exhibits stronger salt tolerance and binding ability to the target molecule, which improves purification efficiency, simplifies the operational process, and meets the urgent need for purifying complex samples in the biopharmaceutical field, providing a more efficient and cost-effective purification solution for the biopharmaceutical industry.

### BRIEF DESCRIPTION OF THE DRAWINGS

FIG. 1 shows a chromatogram of separation of a standard protein using a chromatography medium of Example 3.
FIG. 2 shows a chromatogram for separation of a standard protein using a chromatography medium of Example 5.
FIG. 3 shows a chromatogram for separation of a standard protein using a chromatography medium of Comparative Example 1.
FIG. 4 shows a chromatogram for separation of a standard protein using a chromatography medium of Comparative Example 2.
FIG. 5 shows a chromatogram for separation of a standard protein using a chromatography medium of Comparative Example 3.
FIG. 6 shows a chromatogram for separation of a standard protein using a chromatography medium of Comparative Example 4.
FIG. 7 shows a chromatogram comparing the separation of the standard protein using chromatography media of Comparative Example 1 and Example 3, respectively.
FIG. 8 shows a chromatogram comparing the separation of the standard protein using chromatography media of Comparative Example 2 and Example 3, respectively.
FIG. 9 shows a chromatogram comparing the separation of the standard protein using chromatography media of Comparative Example 3 and Example 3, respectively.
FIG. 10 shows a chromatogram comparing the separation of the standard protein using chromatography media of Comparative Example 4 and Example 3, respectively.
FIG. 11 is a graph showing the salt tolerance test results for chromatography media of Example 3 and Comparative Example 1 respectively, using BSA as a sample.
FIG. 12 is a chromatogram of purification of an actual sample using the chromatography medium of Example 3 in bind-and-elute mode.
FIG. 13 is a chromatogram of purification of an actual sample using the chromatography medium of Comparative Example 1 in bind-and-elute mode.
FIG. 14 is a chromatogram of purification of an actual sample using the chromatography medium of Comparative Example 2 in bind-and-elute mode.
FIG. 15A is a sodium dodecyl sulfate-polyacrylamide gel electrophoresis (SDS-PAGE) image showing different stages of purification of an actual sample using the chromatographic medium of Example 3, wherein M: Marker, 1: loaded sample, 2-11: samples collected from the elution peak every 1 column volume (CV) in Example 3 (a total of 10 CV);
FIG. 15B is an SDS-PAGE image showing different stages of purification of an actual sample using the chromatographic medium of Comparative Example 2, wherein M: Marker, 12-21: samples collected from the elution peak every 1 CV in Comparative Example 2 (a total of 10 CV);
FIG. 15C is an SDS-PAGE image showing different stages of purification of an actual sample using the chromatographic medium of Comparative Example 1, wherein M: Marker, 22-27: samples collected from the elution peak every 0.5 CV in Comparative Example 1 (a total 3 of CV); 28: final 1 CV sample of the elution peak in Comparative Example 1, 29: regenerated sample of Comparative Example 2, and 30: regenerated sample of Example 3.
FIG. 16 shows a liquid-phase detection diagram of sample purification using the chromatography medium of Example 3 in bind-and-elute mode.
FIG. 17 shows a liquid-phase detection diagram of sample purification using the chromatography medium of Comparative Example 1 in bind-and-elute mode.
FIG. 18 shows a liquid-phase detection diagram of sample purification using the chromatography medium of Comparative Example 2 in bind-and-elute mode.
FIG. 19 shows a chromatogram of sample purification using the chromatography medium of Example 3 in flow-through mode.
FIG. 20 shows a chromatogram of sample purification using the chromatography medium of Comparative Example 1 in flow-through mode.
FIG. 21 shows a chromatogram of sample purification using the chromatography medium of Comparative Example 2 in flow-through mode.
FIG. 22 shows a liquid-phase detection diagram of sample purification using the chromatography medium of Example 3 in flow-through mode.
FIG. 23 shows a liquid-phase detection diagram of sample purification using the chromatography medium of Comparative Example 1 in flow-through mode.
FIG. 24 shows a liquid-phase detection diagram of sample purification using the chromatography medium of Comparative Example 2 in flow-through mode.

In the figures, the curve labeled UV-280 represents the absorbance signal detected at an ultraviolet wavelength of 280 nm, also known as the UV280 absorbance curve. The curve labeled UV-400 represents the absorbance signal detected at an ultraviolet wavelength of 400 nm, also known as the UV400 absorbance curve. The curve labeled "Conc" represents the preset volume percentage concentration of the salt-containing buffer solution, also known as the concentration curve. The curve labeled "Cond" represents a conductivity curve. The axis label "Cond (ms/cm)" is an abbreviation for Conductivity (milli Siemens per centimeter).

### DETAILED DESCRIPTION

In order to make the objectives, features, and advantages of the present application more apparent and understandable, detailed explanations of specific embodiments of the present application are provided below with reference to the accompanying drawings. Many specific details are disclosed in the following description to facilitate a comprehensive understanding of the present application. However, the present application can be implemented in various ways different from those described herein, and those skilled in the art may make similar improvements without departing from the scope of the present application. Therefore, the present application is not limited to the specific embodiments disclosed below.

Unless defined otherwise, all technical and scientific terms used herein have the same meaning as commonly understood by one of ordinary skill in the art to which the present application belongs. The terminology used herein in the description of the present application is for the purpose of describing particular embodiments only and is not intended to limit the present application.

Unless otherwise specified, materials, reagents, etc. used in the following examples can be purchased commercially.

As used herein, the term "mixed-mode anion-exchange chromatography medium" refers to a chromatography medium that combines both hydrophobic and ion exchange properties.

As used herein, the terms "matrix carrier" and "matrix microsphere" have the same meaning and refer to a carrier material to be coupled with a ligand. The carrier material can be an inorganic material such as an inorganic polymer and an inorganic silicone material, or an organic material such as a polysaccharide material made from one or more of agarose, cellulose, and dextran, with the molecular weight of dextran ranging from 10 kD to 100 kD.

As used herein, the terms "chromatography medium" and "filler" have the same meaning and refer to a material used for separation and purification.

As used herein, the terms "aggregate" and "multimer" have the same meaning and refer to a complex composed of two or more proteins and formed by the aggregation of monomeric proteins.

In the present application, the terms "chromatographic column" and "chromatography column" have the same meaning and both refer to a column containing a chromatography medium.

In a first aspect, the present application provides a mixed-mode anion-exchange chromatography medium. The chromatography medium includes a ligand coupled to a matrix carrier. The structure of the ligand is represented by R1-R2-N(R3)-R4.

R1 is a substituted or unsubstituted phenyl group. A substituent group of the substituted phenyl group is one or more selected from the group consisting of CH₃, CH₂CH₃, and CH₂CH₂CH₃.

R2 is a substituted or unsubstituted hydrocarbon chain containing 2 to 6 carbon atoms. A substituent group of the substituted hydrocarbon chain is one or more selected from the group consisting of O, CH₃, and CH₂CH₃.

R3 is hydrogen or a hydrocarbon chain containing 1 to 2 carbon atoms.

R4 is hydrogen or a hydrocarbon chain containing 1 to 2 carbon atoms.

The ligand is coupled to the matrix carrier via an amino group.

In some embodiments, R3 and R4 are each hydrogen.

In some embodiments, R3 is a hydrocarbon chain containing 1 to 2 carbon atoms, and/or R4 is a hydrocarbon chain containing 1 to 2 carbon atoms. For example, R3 is CH₃, and R4 is CH₂CH₃; R3 is CH₂CH₃, and R4 is CH₃; R3 and R4 are each CH₂CH₃; R3 is CH₃, and R4 is H; R3 is H, and R4 is CH₃; etc.

In some embodiments, R3 is CH₃, and/or R4 is CH₃. For example, R3 and R4 are each CH₃; R3 is H, and R4 is CH₃; or R3 is CH₃, and R4 is H.

In some embodiments, R2 is an unsubstituted hydrocarbon chain containing 2 to 6 carbon atoms.

In some embodiments, R2 is CH₂CH₂.

In some embodiments, R1 is an unsubstituted phenyl group.

In some embodiments, the ligand is N,N-dimethyl-2-phenylethylamine or a salt thereof.

It should be understood that, when the salt is used as the ligand, the protonated portion of the salt, which is in a cationic form, serves as the required functional portion of the ligand.

In some embodiments, R2 is a substituted hydrocarbon chain containing 2 to 6 carbon atoms.

In some embodiments, R2 is a substituted hydrocarbon chain containing 3 carbon atoms.

In some embodiments, hydrogen on a carbon atom bonded to R1 is substituted with an oxygen atom to form a C=O group.

In some embodiments, the ligand is 3-(dimethylamino) phenylacetone or a salt thereof.

It should be understood that, when the salt is used as the ligand, the protonated portion of the salt, which is in a cationic form, serves as the required functional portion of the ligand for coupling to the matrix carrier.

In some embodiments, the matrix carrier includes a polysaccharide-based matrix carrier. The polysaccharide-based matrix carrier can be selected from corresponding products of VDO Biotech Co., Ltd. (Suzhou, China) or corresponding products commercially available from other manufacturers.

In a second aspect, the present application provides a chromatographic column. The chromatographic column includes the mixed-mode anion-exchange chromatography medium as described above.

In a third aspect, the present application provides use of the above-described chromatography medium or the above-described chromatographic column in purifying a biomolecule.

In some embodiments, the biomolecule is a protein.

In some embodiments, the protein is an antibody, optionally, the antibody includes a monomer and an aggregate.

In a fourth aspect, the present application provides a method for purifying a biomolecule. The method includes: contacting a sample to be purified with the mixed-mode anion-exchange chromatography medium as described above or the chromatographic column as described above; and performing an elution to remove impurities and then collecting a purified target molecule, or purifying the target molecule using a flow-through mode.

The present application will be described in detail below with reference to specific examples. Those examples and comparative examples are provided for purposes of understanding rather than limitation of the present application.

The chromatographic media were made with N,N-dimethyl-2-phenylethylamine as the ligands in the following Examples 1 to 4.

### Example 1

(1) 200 g of agarose-cellulose matrix microspheres (VDO Biotech Co., Ltd. (Suzhou, China), model: Focurose 5HF) were washed with deionized water and added to a 500 mL reaction flask, and 20 g of water, 20 g of anhydrous sodium sulfate, and 40 g of 10 mol/L sodium hydroxide solution were then added. The mixture was stirred until uniform. Subsequently, 0.2 g of sodium borohydride was added and then stirred at 50 °C for 40 min to 60 min. 20 g of allyl glycidyl ether was added as a spacer arm and then stirred at 50 °C for 16 h to 20 h. The system was subjected to suction filtration and washed with deionized water to obtain intermediate microspheres modified with allyl groups on their surfaces.
   The agarose-cellulose matrix microspheres in this example were prepared as follows. (a) 12.5 g of agarose and 250 g of water were weighed and added to a 500 mL reactor, and heated at 98 °C for approximately 6 h to form an agarose solution. In an emulsification vessel, 375 mL of toluene and 8 g of hydroxyethyl cellulose were added and mixed at 60 °C for 0.5 h to 2 h. Subsequently, the agarose solution was transferred into the emulsification vessel, and the rotation speed of the stirrer was gradually increased from 200 r/min to 500 r/min. When the agarose particles were observed under a microscope to have a particle size of 100 µm to 150 µm, the rotation speed was reduced to 200 r/min The reaction solution was then cooled to solidify the agarose particles, and the resulting microspheres were washed with ethanol and water. (b) 100 g of the washed microspheres from step (a) were weighed and added to a 250 mL reactor, followed by the addition of 100 g of water, 10 g of sodium sulfate, 2 mL of 50 wt% sodium hydroxide solution, and 0.1 g of sodium borohydride, and then heated to 40 °C under stirring at the rotation speed of 200 r/min. After that, 30 g of epichlorohydrin and 25 g of 50 wt% sodium hydroxide solution were added dropwise over 7 h. after completion of the dropwise addition, a reaction was carried out at 50 °C for 12 h to 18 h. The reaction mixture was then cooled and washed with water to obtain the agarose-cellulose microspheres Focurose 5HF.
(2) 100 g of the microspheres modified with allyl groups were added to a 250 mL reaction flask. 20 g of deionized water was added and stirred to mix uniformly. 2 g of sodium acetate was added and stirred for 30 min. 3 g of bromine water was added until the color of the mixture turned yellow, and stirred for 10 min. 0.8 g of sodium formate was added to restore the color of the mixture to white, and stirred for 10 min. The system was subjected to suction filtration and washed with deionized water to obtain brominated microspheres.
(3) 100g of the above brominated microspheres were added to a 250 mL reaction flask. 20 g of deionized water was added and stirred to mix uniformly. 1 g of N,N-dimethyl-2-phenylethylamine as a ligand was added. 1 g of 10 mol/L sodium hydroxide solution was added to adjust the pH of the mixture to 11 to 12. 0.15 g of sodium borohydride was added and stirred at 30 °C for 20 h. The resulting mixture was subjected to suction filtration and washed with deionized water to obtain a mixed-mode anion-exchange chromatography medium.

### Example 2

(1) 200 g of agarose matrix microspheres (VDO Biotech Co., Ltd. (Suzhou, China), model: Focurose 6FF) were washed with deionized water and added to a 500 mL reaction flask, and 40 g of water, 40 g of anhydrous sodium sulfate, and 100 g of 10 mol/L sodium hydroxide solution were then added. The mixture was stirred until uniform. Subsequently, 0.4 g of sodium borohydride was added and then stirred at 50 °C for 60 min. 60 g of allyl glycidyl ether was added as a spacer arm and then stirred at 50 °C for 16 h to 20 h. The system was subjected to suction filtration and washed with deionized water to obtain intermediate microspheres modified with allyl groups on their surfaces.
(2) 100 g of the microspheres modified with allyl groups were added to a 250 mL reaction flask. 40 g of deionized water was added and stirred to mix uniformly. 5 g of sodium acetate was added and stirred for 30 min. 5 g of bromine water was added until the color of the mixture turned yellow, and stirred for 10 min. 0.9 g of sodium formate was added to restore the color of the mixture to white, and stirred for 10 min. The system was subjected to suction filtration and washed with deionized water to obtain brominated microspheres.
(3) 100g of the brominated microspheres were added to a 250 mL reaction flask. 40 g of deionized water was added and stirred to mix uniformly. 5 g of N,N-dimethyl-2-phenylethylamine as a ligand was added. 15 g of 10 mol/L sodium hydroxide solution was added to adjust the pH of the mixture to 11 to 12. 0.3 g of sodium borohydride was added and stirred at 30 °C for 20 h. The resulting mixture was subjected to suction filtration and washed with deionized water to obtain a mixed-mode anion-exchange chromatography medium.

### Example 3

(1) 200 g of agarose-cellulose-dextran matrix microspheres (which were prepared with reference to Example 5 of Patent document CN202410328433.3, with the molecular weight of used dextran being 40 kD) were washed with deionized water and added to a 500 mL reaction flask, and 50 g of water, 50 g of anhydrous sodium sulfate, and 200 g of 10 mol/L sodium hydroxide solution were then added. The mixture was stirred until uniform. Subsequently, 0.5 g of sodium borohydride was added and then stirred at 50 °C for 60 min. 100 g of allyl glycidyl ether was added as a spacer arm and then stirred at 50 °C for 16 h to 20 h. The system was subjected to suction filtration and washed with deionized water to obtain intermediate microspheres modified with allyl groups on their surfaces.
(2) 100 g of the microspheres modified with allyl groups were added to a 250 mL reaction flask. 100 g of deionized water was added and stirred to mix uniformly. 8 g of sodium acetate was added and stirred for 30 min. 8 g of bromine water was added until the color of the mixture turned yellow and was stirred for 10 min. 1 g of sodium formate was added to restore the color of the mixture to white and stirred for 10 min. The system was subjected to suction filtration and washed with deionized water to obtain brominated microspheres.
(3) 100g of the brominated microspheres were added to a 250 mL reaction flask. 100 g of deionized water was added and stirred to mix uniformly. 10 g of N,N-dimethyl-2-phenylethylamine as a ligand was added. 30 g of 10 mol/L sodium hydroxide solution was added to adjust the pH of the mixture to 11 to 12. 0.5 g of sodium borohydride was added and stirred at 30 °C for 20 h. The resulting mixture was subjected to suction filtration and washed with deionized water to obtain a mixed-mode anion-exchange chromatography medium.

### Example 4

(1) 200 g of agarose matrix microspheres (VDO Biotech Co., Ltd. (Suzhou, China), model: Focurose 6FF) were washed with deionized water and added to a 500 mL reaction flask, and 60 g of water, 60 g of anhydrous sodium sulfate, and 200 g of 10 mol/L sodium hydroxide solution were then added. The mixture was stirred until uniform. Subsequently, 0.6 g of sodium borohydride was added and then stirred at 50 °C for 60 min. 120 g of allyl glycidyl ether was added as a spacer arm and then stirred at 50 °C for 16 h to 20 h. The system was subjected to suction filtration and washed with deionized water to obtain intermediate microspheres modified with allyl groups on their surfaces.
(2) 100 g of the microspheres modified with allyl groups were added to a 250 mL reaction flask. 100 g of deionized water was added and stirred to mix uniformly. 10 g of sodium acetate was added and stirred for 30 min. 10 g of bromine water was added until the color of the mixture turned yellow and stirred for 10 min. 1.2 g of sodium formate was added to restore the color of the mixture to white, and stirred for 10 min. The system was subjected to suction filtration and washed with deionized water to obtain brominated microspheres.
(3) 100g of the brominated microspheres were added to a 250 mL reaction flask. 120 g of deionized water was added and stirred to mix uniformly. 12 g of N,N-dimethyl-2-phenylethylamine as a ligand was added. 35 g of 10 mol/L sodium hydroxide solution was added to adjust the pH of the mixture to 11 to 12. 0.7 g of sodium borohydride was added and stirred at 30 °C for 20 h. The resulting mixture was subjected to suction filtration and washed with deionized water to obtain a mixed-mode anion-exchange chromatography medium.

Binding capacity is a crucial performance indicator of the chromatography medium. It was evaluated whether the chromatography media prepared in Examples 1 to 4 could meet the minimum binding capacity required for sample purification (generally, a binding capacity ≥ 60 mg bovine serum albumin (BSA)/mL of chromatography medium). The dynamic binding capacity was measured, and the specific conditions were as follows:
1. Chromatography column specification: 1 mL pre-packed column.
2. Determination of dynamic binding capacity with protein

### 2.1 Sample pretreatment

500 mg of BSA was taken, dissolved in equilibration buffer after mixing, and adjusted to a volume of 100 mL. The solution was filtered through a 0.45 µm membrane for later sample loading.

### 2.2 Buffer

Buffer A: 20 mM Bis-Tris, pH 6.5;
Buffer B: 20 mM Bis-Tris, 1 M NaCl, pH 6.5.

The above solutions were filtered through a 0.22 µm membrane prior to use.

### 2.3 Purification process

| | |
|---|---|
| Equilibration | Buffer A, 10 CV; 1 mL/min |
| Sample loading | 0.5mL/min |
| Impurity washing | Buffer A, 20 CV; 1 mL/min |
| Elution | 100% Buffer B, 30 CV; 0.5 mL/min |

The dynamic binding capacities of Examples 1 to 4 are shown in Table 1. As can be seen from Table 1, with an increase in ligand amounts, the dynamic binding capacities of the examples gradually increased. When the ligand amount reached 5 g/100 g of brominated microspheres, the binding capacity of the chromatography medium can meet the binding capacity requirements of conventional chromatography media. When the ligand amount reached 10 g/100 g of brominated microspheres, the binding capacity increased compared with that at 5 g/100 g of brominated microspheres. Furthermore, when the ligand amount was further increased to 12 g/100 g of brominated microspheres, the change in binding capacity was relatively small, indicating that the ligand density coupled onto the matrix carrier gradually tended to stabilize. Further, with all other conditions in Example 1 and Example 2 kept unchanged and only the ligand amount adjusted to 10 g/100 g of brominated microspheres, the prepared chromatography media all exhibited binding capacities greater than 80 mg BSA/mL of chromatography medium. Therefore, a ligand amount of 10 g/100 g of brominated microspheres was selected as the optional amount.

**Table 1**

| Example | IC(µmol/mL) | Binding capacity (mg BSA/mL of chromatography medium) | Recovery rate % |
|---|---|---|---|
| Example 1 | 51.3 | 44.71 | 96.59 |
| Example 2 | 94.9 | 67.38 | 97.62 |
| Example 3 | 118.0 | 82.93 | 93.66 |
| Example 4 | 120.3 | 85.63 | 95.43 |

### Example 5

(1) 200 g of agarose-cellulose-dextran matrix microspheres (which were prepared with reference to Example 6 of Patent CN202410328433.3, with the molecular weight of used dextran being 70 kD) were washed with deionized water and added to a 500 mL reaction flask, and 50 g of water, 50 g of anhydrous sodium sulfate, and 300 g of 10 mol/L sodium hydroxide solution were added. The mixture was stirred until uniform. Subsequently, 0.5 g of sodium borohydride was added and then stirred at 50 °C for 60 min. 150 g of allyl glycidyl ether was added as a spacer arm and then stirred at 50 °C for 16 h to 20 h. The system was subjected to suction filtration and washed with deionized water to obtain intermediate microspheres modified with allyl groups on their surfaces.
(2) 100 g of the microspheres modified with allyl groups were added to a 250 mL reaction flask. 100 g of deionized water was added and then stirred to mix uniformly. 8 g of sodium acetate was added and then stirred for 30 min. 8 g of bromine water was added until the color of the mixture turned yellow, and then stirred for 10 min. 1 g of sodium formate was added to restore the color of the mixture to white, and then stirred for 10 min. The system was subjected to suction filtration and washed with deionized water to obtain brominated microspheres.
(3) 100g of the above brominated microspheres were added to a 250 mL reaction flask. 100 g of deionized water was added and stirred to mix uniformly. 10 g of 3-(dimethylamino)propiophenone as a ligand was added. 30 g of 10 mol/L sodium hydroxide solution was added to adjust the pH of the mixture to 11 to 12. 0.5 g of sodium borohydride was added and stirred at 30 °C for 20 h. The system was subjected to suction filtration and washed with deionized water to obtain a mixed-mode anion-exchange chromatography medium.

In Example 5, 3-(dimethylamino)propiophenone was used as the ligand, with a ligand amount of 10 g. The binding capacity of the chromatography medium prepared in Example 5 was determined using the dynamic binding capacity test method described above. The results are shown in Table 2. As seen from Table 2, the binding capacity of Example 5 is slightly higher than that of Example 3, and it also meets the binding capacity requirement.

**Table 2**

| Example | IC (µmol/mL) | Binding capacity (mg BSA/mL of chromatography medium) | Recovery rate % |
|---|---|---|---|
| Example 5 | 119.3 | 89.86 | 94.88 |

### Comparative Example 1

Comparative Example 1 differed from Example 3 in that: the ligand used in Comparative Example 1 was trimethylamine hydrochloride.

### Comparative Example 2

Comparative Example 2 differed from Example 3 in that: the ligand used in Comparative Example 2 was N-benzyl-N-methyl ethanolamine.

### Comparative Example 3

Comparative Example 3 differed from Example 3 in that: the ligand used in Comparative Example 3 was N,N-dimethylbenzylamine.

### Comparative Example 4

Comparative Example 4 differs from Example 3 in that: the ligand used in Comparative Example 4 was 2,3-epoxypropyltrimethylammonium chloride, and the method for coupling this ligand onto the microspheres differed from the method used in Example 3, without the steps of the allyl group modification and the bromination reaction. The specific method for coupling this ligand was as follows: 200 g of agarose-cellulose-dextran matrix microspheres were reacted with 20 g of 2,3-epoxypropyltrimethylammonium chloride ligand under catalytic conditions, using the ring-opening reaction principle. The epoxy group of 2,3-epoxypropyltrimethylammonium chloride directly coupled with the hydroxyl group on the matrix microspheres. This coupling method is an existing method and will not be described in detail.

The binding capacities of the chromatography media prepared in Comparative Examples 1 to 4 were determined according to the dynamic binding capacity test method described above, and the results are shown in Table 3. As can be seen from Table 3, among Comparative Examples 1 to 4, Comparative Examples 1, 2, and 4 can meet the binding capacity requirements of conventional chromatography media, whereas Comparative Example 3, due to its relatively low binding capacity, is generally not selected as an optional chromatography medium in practical applications.

**Table 3**

| Example | IC (µmol/mL ) | Binding capacity (mg BSA/mL of chromatography medium) | Recovery rate % |
|---|---|---|---|
| Comparative Example 1 | 225.4 | 62.36 | 90.29 |
| Comparative Example 2 | 110.2 | 77.17 | 89.62 |
| Comparative Example 3 | 73.6 | 40.32 | 94.50 |
| Comparative Example 4 | 180.5 | 112.3 | 93.56 |

### (1) Binding Strength Test

In order to further evaluate the binding strengths of the chromatography media prepared in Example 3, Example 5, and Comparative Examples 1 to 4, binding strength testing was performed. Four standard proteins were selected as a mixed sample, and the elution times of different proteins were measured under 0-1 M NaCl concentration conditions. The specific test conditions were as follows:
1. Chromatography Column Specification: 1 mL pre-packed column.
2. Measurement of Standard Protein Retention Time

### 2.1 Sample Pretreatment

7 mg of Lysozyme, 15 mg of Myoglobin, 100 mg of Albumin, and 100 mg of Trypsin Inhibitor were taken and mixed, dissolved in equilibration buffer, and adjusted to a volume of 10 mL. The solution was filtered through a 0.45 µm membrane for later sample loading.

### 2.2 Buffer

Buffer A: 20 mM Bis-Tris, pH 6.5;
Buffer B: 20 mM Bis-Tris, 1 M NaCl, pH 6.5;

The above solutions were filtered through a 0.22 µm membrane before use.

### 2.3 Purification Process

| | |
|---|---|
| Equilibration | Buffer A, 10 CV; 1 mL/min |
| Sample loading | 0.5mL/min; 250µL |
| Elution | 0-100% Buffer B, 30 CV; 100% Buffer B, 5CV; 0.5mL/min |

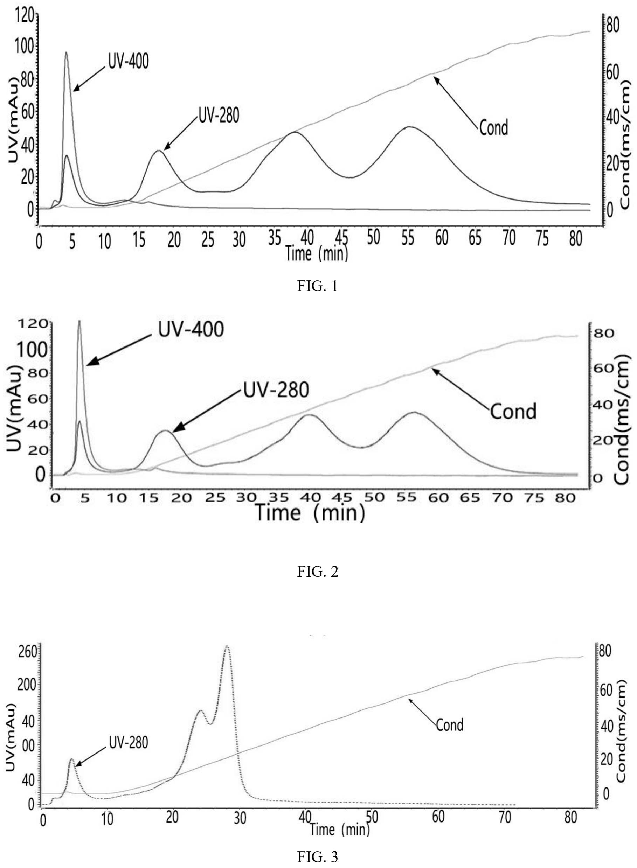

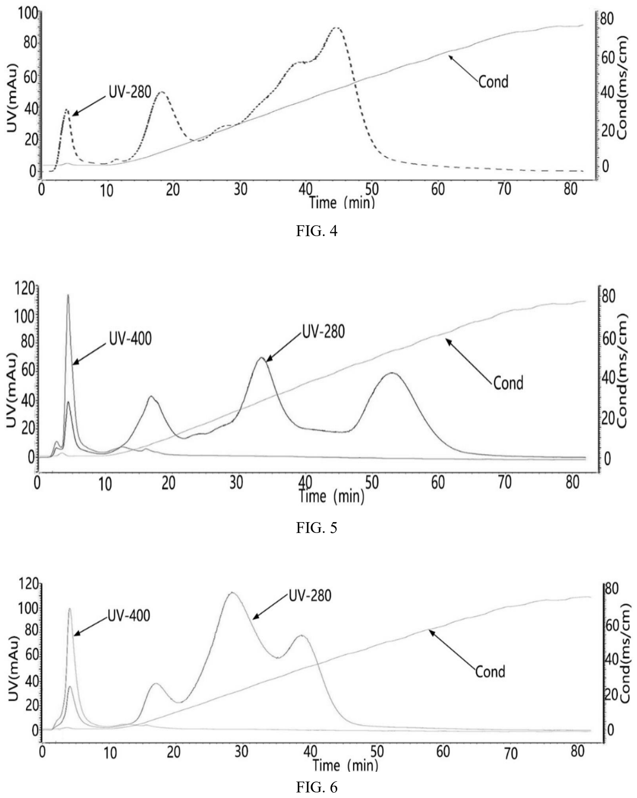

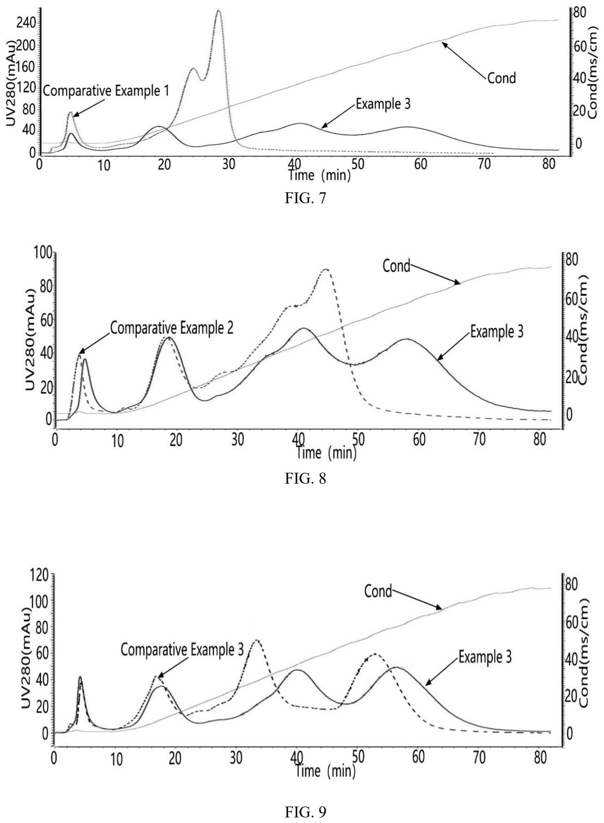

The chromatograms of Example 3, Example 5, and Comparative Examples 1 to 4 are shown in FIGS. 1 to 6, respectively. FIGS. 7 to 10 respectively show chromatograms in which the results of Comparative Examples 1 to 4 were each overlaid with the result of Example 3, thereby clearly demonstrating the differences in retention times between the comparative examples and the example. As can be seen from these figures, the protein retention times corresponding to Example 3 and Example 5 are basically consistent, whereas the protein retention times of Comparative Examples 1 to 4 are all shorter than the corresponding retention times of Example 3, indicating that the protein binding abilities of the chromatography media of Example 3 and Example 5 are significantly stronger than those of Comparative Examples 1 to 4, and indirectly reflecting that the ligands N,N-dimethyl-2-phenylethylamine and 3-(dimethylamino)propiophenone exhibit higher salt tolerance than the ligands trimethylamine hydrochloride, N-benzyl-N-methyl ethanolamine, N,N-dimethylbenzylamine, and 2,3-epoxypropyltrimethylammonium chloride.

Based on the preparation method of Example 3, the matrix microspheres in Example 3 were replaced with agarose microspheres and agarose-cellulose microspheres, respectively, and then the binding strength of the two prepared chromatography media was tested under the above conditions. The obtained chromatograms were comparable to the results obtained in Example 3, indicating that the type of matrix microsphere does not affect the protein binding strength of the chromatography media prepared with such ligand.

### (2) Salt Tolerance Test

The salt tolerance of the chromatography media was evaluated by analyzing the influence of different conductivity conditions on the BSA binding capacity of different chromatography media. The test method was as follows.
1. Chromatography Column Specification: 1 mL pre-packed column.
2. Measurement of Dynamic Protein Binding Capacity

### 2.1 Sample Pretreatment

500 mg of BSA was taken, dissolved in equilibration buffers with different conductivities after mixing, and adjusted to a volume of 100 mL. The solution was filtered through a 0.45 µm membrane for later sample loading.

### 2.2 Buffer

Buffer A: 20mM Tris, X mM NaCl, pH 8.5, wherein the values of X are 0, 40, 80, 160, etc.;
Buffer B: 20mM Tris, 1 M NaCl, pH 8.5;

The above solutions were filtered through a 0.22 µm membrane before use.

### 2.3 Purification Process

| | |
|---|---|
| Equilibration | Buffer A, 10 CV; 1 mL/min |
| Sample loading | 0.5mL/min; 25mL |
| Impurity washing | Buffer A, 20 CV; 1 mL/min |
| Elution | 100% Buffer B, 30 CV; 0.5mL/min |

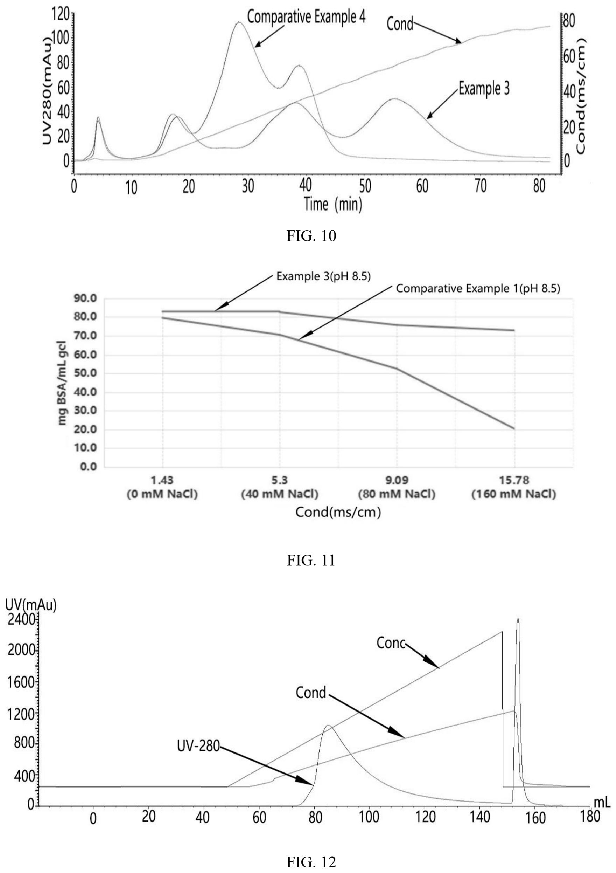

First, the salt tolerances of the chromatography media of Example 3 and Comparative Example 1 were tested. The test results are shown in FIG. 11. As can be seen from FIG. 11, as the conductivity increased, the binding capacity of the chromatography medium of Example 3 for the BSA sample remained consistently higher than that of Comparative Example 1, indicating that the chromatography medium of Example 3 exhibited better salt tolerance than that of Comparative Example 1.

Subsequently, the salt tolerances of the chromatography media of Example 5, Comparative Example 2, and Comparative Example 4 were tested under the same conditions described above. The test result of Example 5 was substantially consistent with the curve of Example 3 shown in FIG. 11, while the test curves of Comparative Example 2 and Comparative Example 4 were both lower than those of Example 3 and Example 5, indicating that the chromatography media of Example 3 and Example 5 exhibited stronger salt tolerance.

### (3) Antibody Sample Purification Test-Bind-and-elute mode

The chromatography media prepared in Example 3, Comparative Example 1, and Comparative Example 2 were each subjected to the antibody sample purification test. The specific testing procedure is as follows.

### 1. Sample Preparation

The CHO cell culture solution was centrifuged and filtered through a 0.22 µm membrane. Then, affinity purification was performed using Protein A chromatography medium, and the crude eluate containing antibodies was collected. The purification process for Protein A affinity chromatography can follow the procedure of commercially available Protein A affinity chromatography media. For example, arProtein A Focurose HR (VDO Biotech Co., Ltd., Suzhou, China) can be used for initial purification. The affinity eluted sample obtained after the initial purification was diluted with 20 mM Tris-HCl buffer (pH 7.2) to a concentration of 4.39 mg/mL. The diluted and affinity eluted sample was adjusted to pH 7.2 using 1M Tris and filtered through a 0.22 µm membrane for later use.

### 2. Chromatography Column and Buffer Preparation

### 2.1 Chromatography Column

The column specifications for the three chromatography media were consistent. The chromatography column had a diameter of 0.8 cm, a height of 10 cm, and a column volume of 5 mL.

### 2.2 Buffer

Equilibration buffer (A): 20 mM Tris-HCl, pH 7.2
Elution buffer (B): 20 mM Tris-HCl, 1 M NaCl, pH 7.2
Regeneration buffer: 1 M HAc
Sanitization buffer: 1 M NaOH

### 3. Chromatography procedure

The three chromatography media were used to purify the same sample according to the same chromatography procedure as follows.

| Step | Buffer | Flow Rate (mL/min) | Column Volume (CV) | Notes |
|---|---|---|---|---|
| Equilibration | 20mM Tris-HCl, pH7.2 | 2 | 4 | The pH and conductivity need to reach a stable state |
| Sample loading | Diluted and pH-adjusted affinity eluate | 1.5 | N/A | Sample volume: 28.5mL |
| Washing | 20mM Tris-HCl, pH7.2 | 1.5 | 4 | The chromatography parameter UV need to reach a flat baseline |
| Elution | Buffer A+buffer B linear elution, 0-100%B, 20CV | 1.5 | 20 | Peak collection range: 50-max-100mAU |
| Regeneration | 1M HAc | 2 | 3 | Peak collection range: 100-max-100mAU |
| Rinsing 1 | 20mM Tris-HCl, pH7.2 | 2 | 4 | N/A |
| Sanitization | 1M NaOH | 2 | 4 | N/A |
| Rinsing 2 | 20mM Tris-HCl, 1M NaCl, pH7.2 | 2 | 4 | N/A |

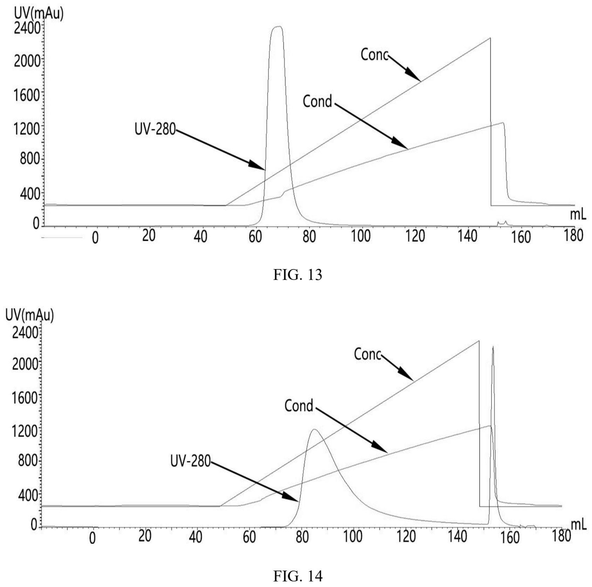

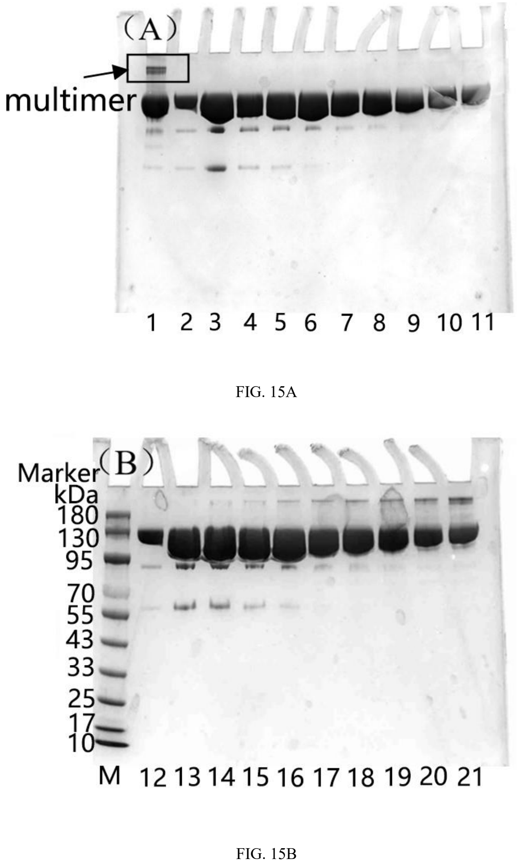

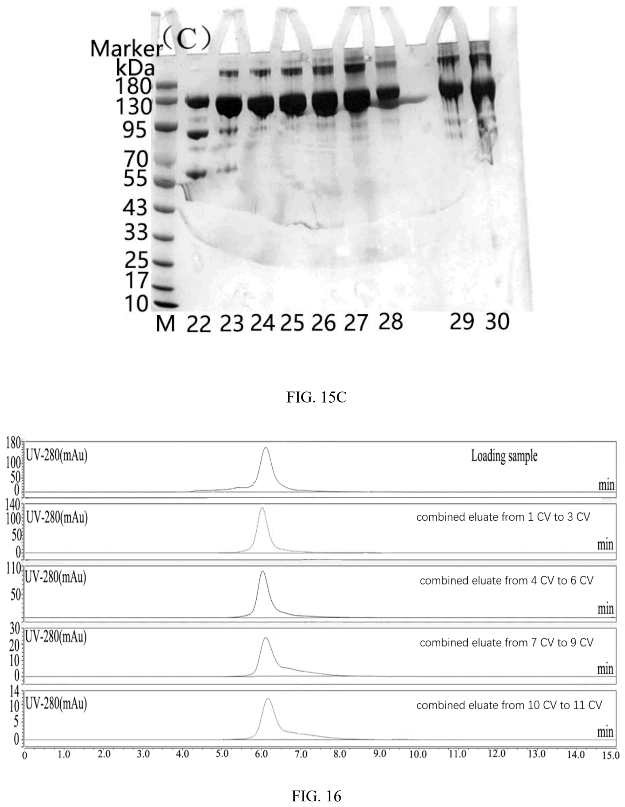

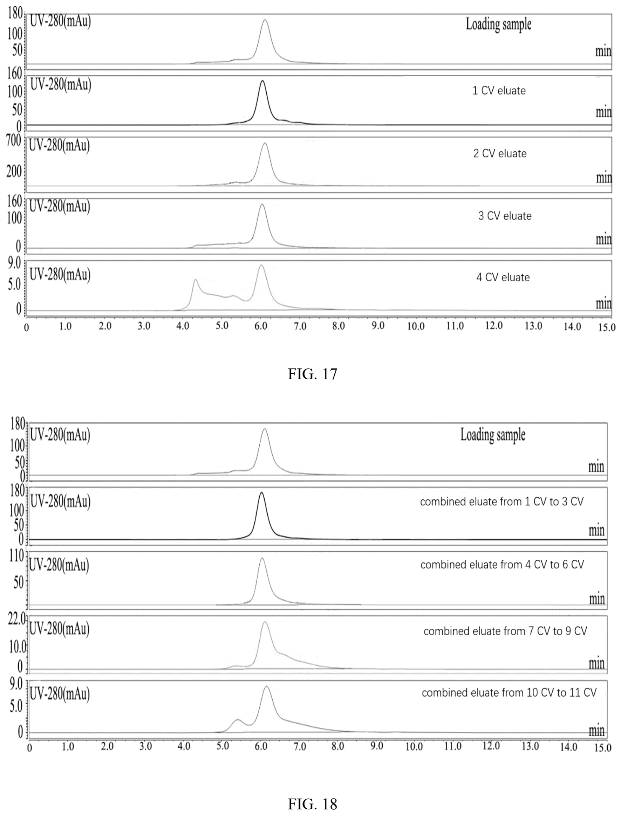

The three fillers were tested using the same sample and buffers. A 0-100% linear gradient elution over 20 column volumes (CV) was applied. The elution peak was fractionated and collected in 1 CV per tube. The chromatograms of the chromatography media are shown in FIGS. 12 to 14, respectively. The collected sample purified with each chromatography medium were subjected to sodium dodecyl sulfate-polyacrylamide gel electrophoresis (SDS-PAGE) detection and size-exclusion chromatography (SEC) liquid-phase detection. The results of SDS-PAGE detection are shown in FIG. 15, and the results of SEC liquid-phase detection are shown in FIGS. 16 to 18, respectively.

For the chromatography medium of Example 3, both the SDS-PAGE image and the liquid-phase detection result graph show that the multimers were not eluted during the 20 CV elution process, and the multimer only dissociated during the regeneration step, indicating that such filler can substantially completely remove the multimers from the antibodies. For the chromatography medium of Comparative Example 1, during the 20 CV elution process, all of the sample bound to the filler was dissociated within the first 4 CV, and it was found through detection that the multimers began to dissociate from the 1st CV, and the entire elution peak consisted of a mixture of multimers and monomers, indicating that such filler showed little to no removal of multimers from the antibodies. For the chromatography medium of Comparative Example 2, during the 20 CV elution process, the monomers were eluted in the first 4 CV, and from the 5th CV, the multimers began to dissociate as well, and the subsequent elution samples were all contained a mixture of multimers and monomers, indicating that such filler showed partial removal of multimers from the antibodies.

### (4) Antibody Sample Purification Test-Flow-through Mode

The chromatography media prepared in Example 3, Comparative Example 1, and Comparative Example 2 were each subjected to the antibody sample purification test. The specific testing procedure is as follows.

### 1. Sample Preparation

The CHO cell culture solution was subjected to initial purification through the same method as described in the above bind-and-elute mode. The affinity eluted sample obtained after the initial purification was diluted with 20mM phosphate buffer (PB) at pH 6.0 and 150mM arginine to a concentration of 4.88 mg/mL. The diluted and affinity eluted sample was adjusted to pH 6.0 using 1M Tris and filtered through a 0.22 µm membrane for later use.

### 2. Chromatography Column and Buffer Preparation

### 2.1 Chromatography Column

The column specifications for the three chromatography media were consistent. The chromatography column had a diameter of 0.8 cm, a height of 10 cm, and a column volume of 5 mL.

### 2.2 Buffer

Equilibration buffer: 20 mM PB, 150mM arginine, pH 6.0;
Regeneration buffer: 20mM PB, 1M NaCl, pH 6.0;
Sanitization buffer: 1 M NaOH.

### 3. Chromatography procedure

The three chromatography media were used to purify the same sample according to the same chromatography procedure as follows.

| Step | Buffer | Flow Rate (mL/min) | Column Volume (CV) | Notes |
|---|---|---|---|---|
| Equilibration | 20 mM PB, 150mM arginine, pH 6.0 | 2 | ≥4 | The pH and conductivity need to reach a stable state |
| Sample loading | Diluted and pH-adjusted affinity eluate | 1.5 | N/A | Peak collection range: 100-max-100mAU |
| Washing | 20 mM PB, 150mM arginine, pH 6.0 | 1.5 | ≥3 | |
| Regeneration | 20mM PB, 1M NaCl, pH 6.0 | 2 | ≥3 | Peak collection range: 40-max-40mAU |
| Sanitization | 1M NaOH | 2 | ≥3 | N/A |
| Rinsing 2 | 20mM PB, 1M NaCl, pH 6.0 | 2 | ≥3 | N/A |

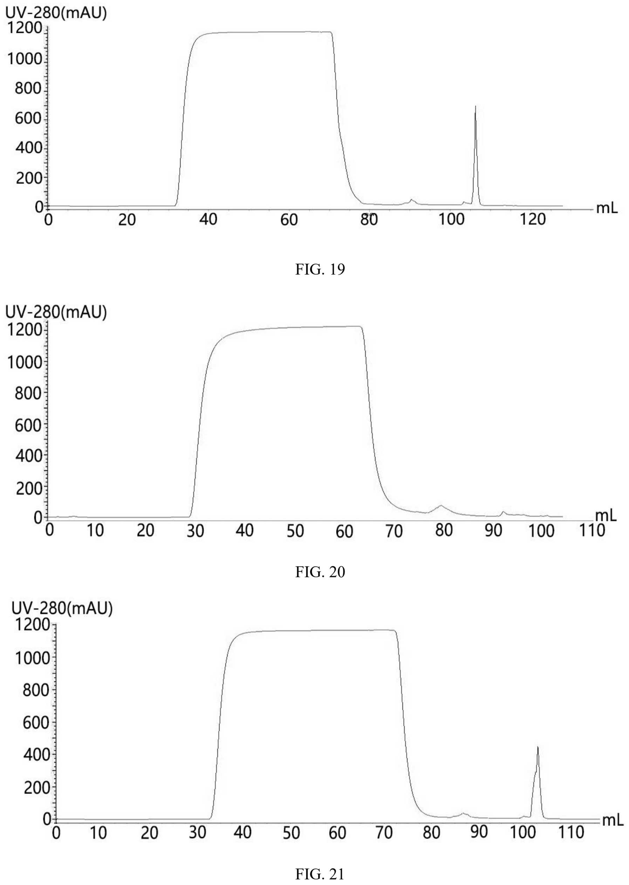

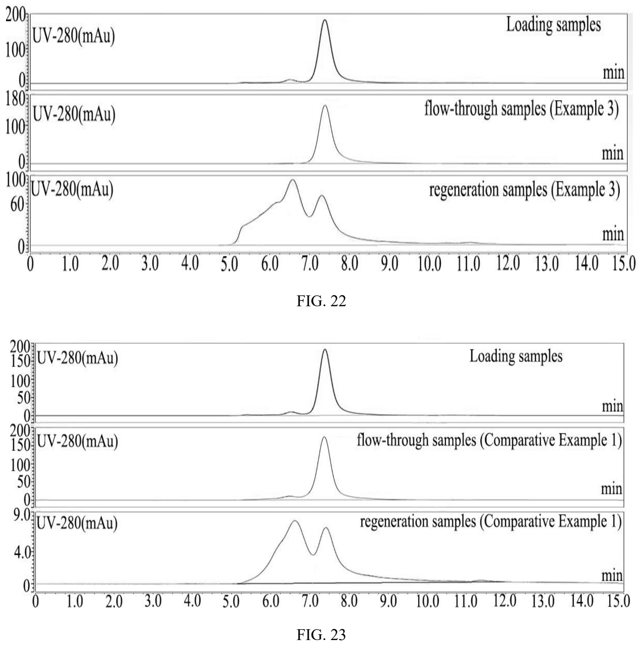

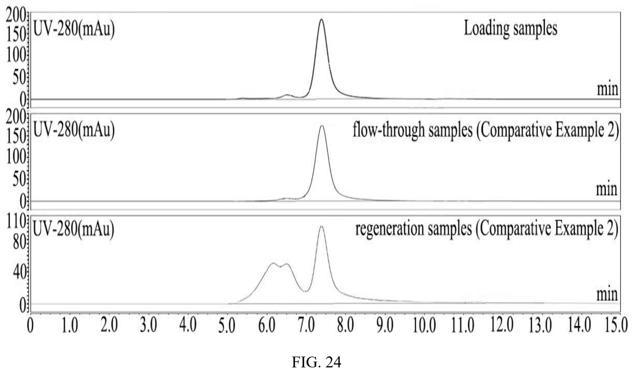

The three fillers were tested using the same sample and buffers, with the chromatography modes being the flow-through mode. The flow-through peak during sample loading and the regeneration peak were collected. The chromatograms for the chromatography media are shown in FIGS. 19 to 21, respectively. The collected sample purified with each chromatography medium were subjected to SEC liquid-phase detection. The results of SEC liquid-phase detection are shown in FIGS. 22 to 24, respectively.

For the chromatography medium of Example 3, the liquid-phase detection result graph shows that the flow-through sample consisted essentially of monomers, whereas the regeneration sample contained a relatively high proportion of multimers, indicating that such filler can substantially completely remove the multimers from the antibodies. For the chromatography medium of Comparative Example 1, the liquid-phase detection result shows that the flow-through sample was a mixture of multimers and monomers, and although both multimers and monomers were present in the regeneration peak, the overall content in the regeneration sample was low, indicating that such filler showed little to no removal of multimers from the antibodies. For the chromatography medium of Comparative Example 2, multimers were partially removed in the flow-through sample but could not be completely removed, and although multimers were present in the regeneration sample, the proportion of monomers remained relatively high, indicating that such filler showed partial removal of multimers from the antibodies.

In summary, through purification tests using actual samples, it has been found that the chromatography medium of Example 3 exhibits excellent separation performance in multimer removal. When an antibody sample containing multimers passes through the chromatography column of Example 3, due to differences in properties such as charge between multimers and monomers, the monomers flow through the filler while the multimers are adsorbed onto the filler during the sample loading by controlling loading conditions, thereby achieving effective separation. Consequently, the chromatography medium of Example 3 shows significantly superior performance of removing multimers from antibodies compared with those of Comparative Examples 1 and 2.

Based on the preparation method of the chromatography medium of Example 3, the matrix microspheres in Example 3 were replaced with agarose microspheres and agarose-cellulose microspheres, respectively, and then the resulting two chromatography media and the chromatography medium of Example 5 were subjected to purification tests using the same purification methods (bind-and-elute mode and flow-through mode) as in Example 3. Those three chromatography media also achieved purification effects comparable to that of Example 3 and achieved separation of monomers and multimers.

## Claims

1. A mixed-mode anion-exchange chromatography medium comprising a ligand coupled to a matrix carrier, a structure of the ligand is represented by R1-R2-N(R3)-R4, wherein:
R1 is a substituted or unsubstituted phenyl group, and a substituent group of the substituted phenyl group is one or more selected from the group consisting of CH₃, CH₂CH₃, and CH₂CH₂CH₃;
R2 is a substituted or unsubstituted hydrocarbon chain containing 2 to 6 carbon atoms, and a substituent group of the substituted hydrocarbon chain is one or more selected from the group consisting of O, CH₃, and CH₂CH₃;
R3 is hydrogen or a hydrocarbon chain containing 1 to 2 carbon atoms;
R4 is hydrogen or a hydrocarbon chain containing 1 to 2 carbon atoms; and
the ligand is coupled to the matrix carrier via an amino group.

2. The mixed-mode anion-exchange chromatography medium according to claim 1, wherein R3 and R4 are each hydrogen.

3. The mixed-mode anion-exchange chromatography medium according to claim 1, wherein R3 is a hydrocarbon chain containing 1 to 2 carbon atoms, preferably, R3 is CH₃, and/or R4 is a hydrocarbon chain containing 1 to 2 carbon atoms, preferably, R4 is CH₃.

4. The mixed-mode anion-exchange chromatography medium according to claim 1, wherein R2 is an unsubstituted hydrocarbon chain containing 2 to 6 carbon atoms, preferably, R2 is CH₂CH₂.

5. The mixed-mode anion-exchange chromatography medium according to any one of claims 1 to 4, wherein R1 is an unsubstituted phenyl group.

6. The mixed-mode anion-exchange chromatography medium according to any one of claims 1 and 3 to 4, wherein the ligand is N,N-dimethyl-2-phenylethylamine or a salt thereof.

7. The mixed-mode anion-exchange chromatography medium according to claim 1, wherein R2 is a substituted hydrocarbon chain containing 2 to 4 carbon atoms.

8. The mixed-mode anion-exchange chromatography medium according to claim 7, wherein R2 is a substituted hydrocarbon chain containing 3 carbon atoms, preferably, hydrogen on a carbon atom bonded to R1 is substituted with an oxygen atom to form a C=O group.

9. The mixed-mode anion-exchange chromatography medium according to any one of claims 7 to 8, wherein R1 is an unsubstituted phenyl group.

10. The mixed-mode anion-exchange chromatography medium according to any one of claims 1, 3, 7, and 8, wherein the ligand is 3-(dimethylamino)propiophenone or a salt thereof.

11. The mixed-mode anion-exchange chromatography medium according to claim 1, wherein the matrix carrier comprises a polysaccharide-based matrix carrier.

12. The chromatographic column, comprising the mixed-mode anion-exchange chromatography medium according to any one of claims 1 to 11.

13. Use of the chromatography medium according to any one of claims 1 to 11 or the chromatographic column according to claim 12 in purifying a biomolecule.

14. The use of claim 13, wherein the biomolecule is a protein, preferably, an antibody.

15. A method for purifying a biomolecule, comprising:
contacting a sample to be purified with the mixed-mode anion-exchange chromatography medium according to any one of claims 1 to 11 or the chromatographic column according to claim 12; and
performing an elution to remove impurities and then collecting a purified target molecule, or purifying the target molecule using a flow-through mode
